(19) Europäisches Patentamt
European Patent Office
Office européen des brevets

(11) EP 2 376 511 B1

(12) # FASCICULE DE BREVET EUROPEEN

(45) Date de publication et mention de la délivrance du brevet:
**12.03.2014 Bulletin 2014/11**

(21) Numéro de dépôt: **09801747.8**

(22) Date de dépôt: **08.12.2009**

(51) Int Cl.:
*C07H 15/24* (2006.01) *C07H 17/00* (2006.01)
*C07H 7/06* (2006.01) *A61K 31/485* (2006.01)
*A61K 31/519* (2006.01) *A61K 31/7042* (2006.01)
*A61P 25/04* (2006.01) *C07D 489/02* (2006.01)
*C07D 487/04* (2006.01) *C07D 249/14* (2006.01)
*C07D 239/18* (2006.01) *A61K 31/7064* (2006.01)

(86) Numéro de dépôt international:
**PCT/FR2009/052446**

(87) Numéro de publication internationale:
**WO 2010/067008 (17.06.2010 Gazette 2010/24)**

(54) **DERIVES BICYCLIQUES DE MORPHINE-6-GLUCURONIDE, LEUR PREPARATION ET LEUR APPLICATION EN THERAPEUTIQUE**

BIZYKLISCHE DERIVATE VON MORPHIN-6-GLUCURONID, VERFAHREN ZU IHRER HERSTELLUNG UND IHRE THERAPEUTISCHE VERWENDUNG

BICYCLIC DERIVATIVES OF MORPHINE-6-GLUCURONID, PROCESS FOR THEIR PREPARATION AND THEIR APPLICATION IN THERAPY

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**
Etats d'extension désignés:
**AL BA RS**

(30) Priorité: **11.12.2008 FR 0806974**

(43) Date de publication de la demande:
**19.10.2011 Bulletin 2011/42**

(73) Titulaire: **SANOFI**
**75008 Paris (FR)**

(72) Inventeurs:
- **DLUBALA, Alain**
  **F-75013 Paris (FR)**
- **RIPOCHE, Isabelle**
  **F63174 Aubière Cedex (FR)**
- **TRECANT, Claire**
  **F-67000 Strasbourg (FR)**

(74) Mandataire: **Veinante, Aude et al**
**Sanofi**
**Département Brevets**
**54, rue La Boétie**
**75008 Paris (FR)**

(56) Documents cités:
**WO-A-98/46618 FR-A- 2 864 082**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 376 511 B1

## Description

## DOMAINE DE L'INVENTION

La présente invention se rapporte à des dérivés bicycliques de morphine-6-glucuronide, à leur préparation et à leur utilisation pour le traitement et la prévention de la douleur.

Les dérivés de morphine-6-glucuronide et les dérivés de morphine-6-glucuronamide sont décrits dans les demandes de brevets WO 98/44618 et FR 2 864 082 en tant qu'analgésiques.

La présente invention a pour objet les composés répondant à la formule (I)

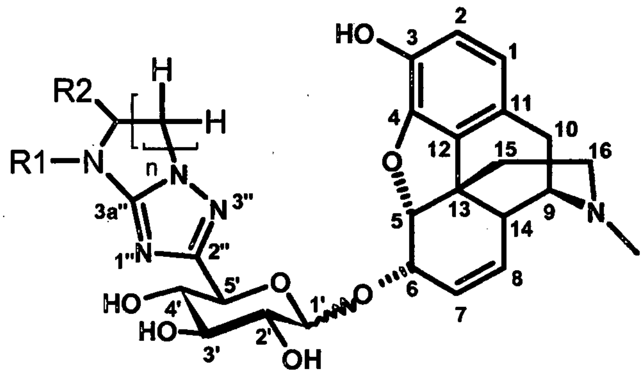

(I)

dans laquelle :

R1 représente un atome d'hydrogène ou un groupe $(C_1\text{-}C_4)$alkyle,
R2 représente un groupe hydroxyle, un groupe thiol, un groupe $(C_1\text{-}C_4)$alkyloxy ou un groupe thio$(C_1\text{-}C_4)$alkyle, et n est un nombre entier égal à 1 ou 2,

à l'état de base ou de sel d'addition à un acide ainsi qu'à l'état d'hydrate ou de solvate.

Les composés de formule (I) peuvent comporter un carbone asymétrique. Ils peuvent donc exister sous forme de deux énantiomères. Ces énantiomères ainsi que leurs mélanges, y compris les mélanges racémiques, font partie de l'invention.

Les composés de formule (I) comportent un carbone anomérique. Ils peuvent exister sous la forme d'anomères $\alpha$ ou $\beta$. Les anomères $\alpha$, $\beta$ et leur mélange font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles par exemple pour la purification ou l'isolement des composés de formule (I) font également partie de l'invention.

Les composés de formule (I) peuvent également exister sous forme d'hydrates ou de solvates, à savoir sous forme d'associations ou de combinaisons avec une ou plusieurs molécules d'eau ou avec un solvant. De tels hydrates et solvates font également partie de l'invention.

Dans le cadre de la présente invention, on entend par :

- un groupe $(C_1\text{-}C_4)$alkyle : un groupe aliphatique saturé, linéaire ou ramifié, substitué ou non substitué, ayant entre 1 et 4 atomes de carbone ; à titre d'exemples, on peut citer les groupes méthyle, éthyle, propyle, isopropyle, butyle, isobutyle et tertbutyle ;
- un groupe hydroxyle : un groupe-OH ;
- un groupe thiol : un groupe -SH ;
- un groupe $(C_1\text{-}C_4)$alkyloxy: un groupe -O-$(C_1\text{-}C_4)$alkyle où le groupe $(C_1\text{-}C_4)$alkyle est tel que précédemment défini ; à titre d'exemples, on peut citer les groupes méthoxy, éthoxy, propoxy et butyloxy ; et
- un groupe thio$(C_1\text{-}C_4)$alkyle: un groupe -S-$(C_1\text{-}C_4)$alkyle où le groupe $(C_1\text{-}C_4)$alkyle est tel que précédemment défini ; à titre d'exemples, on peut citer les groupes thiométhyle, thioéthyle, thiopropyle et thiobutyle.

Parmi les composés de formule (I) selon l'invention, un premier groupe de composés présentent une ou plusieurs

des caractéristiques suivantes:

- R1 est un atome d'hydrogène,
- R2 est un groupe hydroxy, et
- n est égal à 2.

Parmi les composés de formule (I) selon l'invention, on peut citer en particulier le composé suivant :

- le morphin-6-yl 5-*C*-(5-hydroxy-4,5,6,7-tétrahydro[1,2,4]triazolo[1,5-*a*]pyrimidin-2-yl)-β-D-xylopyranoside.

**PROCEDE DE PREPARATION**

Dans ce qui suit, on entend par groupe protecteur, un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 2nd Edition (John Wiley & Sons, Inc., New York). On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 3rd Edition, Wiley Interscience, p. 310-316.

Le schéma réactionnel 1 ci-dessous illustre la réaction pour l'exemple particulier du morphin-6-yl 5-*C*-(5-hydroxy-4,5,6,7-tétrahydro[1,2,4]triazolo[1,5-*a*]pyrimidin-2-yl)-β-D-xylopyranoside.

Schéma réactionnel 1

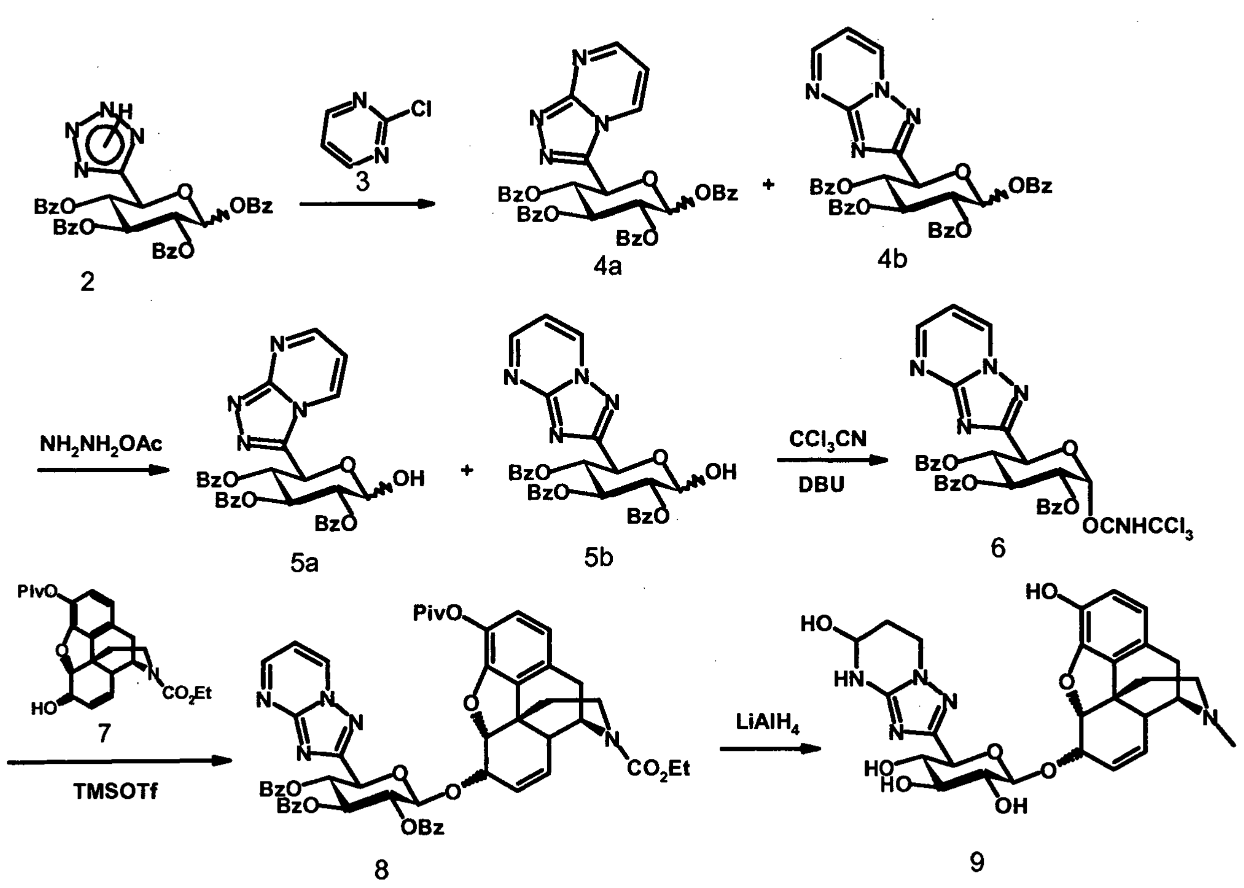


Dans une première étape, le composé (7), portant un groupe protecteur en position 3 du cycle morphinique

par exemple un groupe pivaloyle et en position 17 par un groupe carboxylate d'éthyle peut être couplé au composé (6) préalablement protégé par exemple par groupe benzoyle et dont la fonction hydroxy du carbone anomérique est activée par exemple par un groupe trichloroacétimidate $-CNHCCl_3$, pour obtenir le composé (8).
La réaction de couplage peut par exemple s'effectuer en présence d'un acide de Lewis, tel que le trifluorométhanesulfonyl de triméthylsilane (TMSOTf), dans un solvant tel que le dichlorométhane, à une température comprise entre 0°C et 25°C.
Le composé (7) peut être préparé par exemple selon la méthode décrite dans Portoghese and coll. J. Med. Chem. 1972, 15, 208-210.
Le composé (6) peut être obtenu par activation de la fonction hydroxyle du composé (5). Dans le cas où le groupe activateur est un groupe trichloroacétimidate $-CNHCCl_3$, la réaction peut se faire en présence de trichloroacétonitrile et d'une base forte telle que le 1,8-diazabicyclo[5.4.0]undec-7-ène, dans un solvant tel que le dichlorométhane.

Le composé (5) peut être préalablement obtenu par déprotection anomérique du composé (4). Par exemple, lorsque le groupe protecteur est un groupe benzoyle, la déprotection du groupe hydroxyle peut se faire en présence d'acétate d'hydrazine (NH2NH2, CH3COOH).

Dans une seconde étape, le composé (8) est réduit et déprotégé simultanément, par exemple en présence d'hydrure de lithium et d'aluminium, dans un solvant tel que le tétrahydrofurane, à la température de reflux du milieu réactionnel, puis isolé en présence d'un acide minéral tel que l'acide chlorhydrique. On obtient ainsi le composé (9) qui est un exemple de composé de formule générale (I).

L'invention est illustrée de manière non limitative par les exemples ci-dessous.

## <u>EXEMPLE</u>

### 1.1. 1,2,3,4-tétra-*O*-benzoyl-α/β-D-glucurononitrile

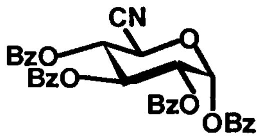

**1a**

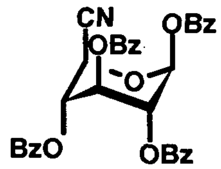

**1b**

A une suspension de D-glucuronamide (25,0 g, 0,129 mol) dans la pyridine (100 mL) à température ambiante, est ajoutée en 30 min une solution de chlorure de benzoyle (102 mL, 0,878 mol) dans du dichlorométhane (90 mL). Le milieu réactionnel est agité une nuit à température ambiante puis du dichlorométhane (200 mL) et de l'eau (200 mL) sont ajoutés. La phase organique est lavée avec une solution d'acide chlorhydrique 1 N (200 mL), une solution saturée d'hydrogénocarbonate de sodium (3 x 200 mL) et une solution saturée de chlorure de sodium (200 mL). La phase organique est séchée sur sulfate de sodium et le solvant éliminé sous pression réduite. Le résidu (huile jaune) est trituré dans l'éthanol (200 mL) pour donner un mélange d'anomères (43,4 g, 57%) sous forme de cristaux de couleur jaune pâle. Le spectre de RMN du proton dans le deutériochloroforme, $CDCl_3$, montre un rapport α:β de 2:1.
Point de fusion : 209-212°C.
RMN $^1H$ (400 MHz, $CDCl_3$) : δ 8,10-7,30 (m, 20Hα+20H β, H-*aro*), 6,88 (d, 1Hα, *J* 3,5 Hz, H-1α), 6,57 (d, 1 H β, *J* 3,0 Hz, H-1 β), 6,21 (t, 1Hα, *J* 9,5 Hz, H-3α), 5,93 (t, 1Hα, *J* 9,5 Hz, H-4α), 5,84 (t, 1 H β, *J* 4,0 Hz, H-3 β), 5,71-5,65 (m, 1Hα+1H β, H-2q , H-4β), 5,64 (m, 1 H β, H-2 β), 5,16 (d, 1 H β, *J* 4,0 Hz, H-5 β), 5,11 (d, 1Hα, *J* 9,5 Hz, H-5).
RMN $^{13}C$ (100 MHz, $CDCl_3$) δ 165,5, 165,1, 164,8, 164,6, 164,3, 163,8 (C=O), 134,4-128,0 (C-*aro*), 115,3 (C-6 β), 114,1 (C-6α), 90,9 (C-1 β), 89,4 (C-1α), 69,3, 69,2, 69,0 (C-2 α, C-3 α, C-4 α), 67,4 (C-4 β), 66,7, 66,5 (C-2 β, C-3 β), 61,9 (C-5 α), 60,8 (C-5 β). Masse calculée pour $C_{34}H_{25}NO_9Na$ $[M+Na]^+$ 614,1427, trouvée 614,1422.

### 1.2. 1,2,3,4-tétra-*O*-benzoyl-5-*C*-(tétrazol-5-yl)-α/β-D-xylopyranose

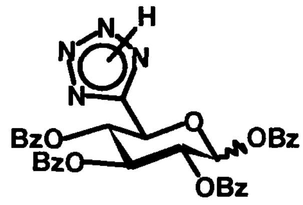

**2**

A une solution de 1,2,3,4-tétra-*O*-benzoyl-α/β-D-glucurononitrile précédemment préparé (43,0 g, 72,8 mmol) dans du toluène (500 mL) sont ajoutés de l'oxyde de bis-(tributylétain) (3,70 mL, 7,26 mmol) et de l'azidure de triméthylsilyle (28,7 mL, 216 mmol). Le milieu réactionnel est agité une nuit à reflux. Le solvant est éliminé sous pression réduite et le résidu est purifié par chromatographie sur gel de silice (cyclohexane-acétate d'éthyle 1:1 à 0:1) pour donner le 1,2,3,4-tétra-*O*-benzoyl-5-*C*-(tétrazol-5-yl)-α/β-D-xylopyranose (27,0 g, 59%) sous forme de cristaux bruns. Le spectre de RMN du proton dans $CDCl_3$ montre un rapport α: β de 2:1.
Point de fusion 144-147°C.
RMN $^1$H (400 MHz, $CDCl_3$) : δ 8,19-7,28 (m, 20Hα+20H β, H-*aro*), 7,06 (d, 1Hα, J 3,5 Hz, H-1α), 6,50-6,44 (m, 1 H β +1Hα, H-1 β. H-3α), 6,21 (t, 1 H β, *J* 9,0 Hz, H-3 β), 6,13-6,01 (m, 2H β. +1Hα, H-4 β, H-4α. H-2 β), 5,90-5,85 (m, 2Hα, H-2α, .H-5α), 5,66 (d, 1 H β, J 9,0 Hz, H-5 β).
RMN $^{13}$C (100 MHz, $CDCl_3$) δ 165,79, 165,2, 164,7 (C=O, C=N), 134,4-128,1 (C-*aro*), 93,0 (C-1 β), 89,9 (C-1α), 72,0, 70,5, 70,4, 70,3, 69,5 (C-2α, C-2 β, C-3α, C-3 β, C-4α, C-4 β), 68,9 (C-5 β), 67,0 (C-5α).
Masse calculée pour $C_{34}H_{26}N_4O_9Na$ $[M+Na]^+$ 657,1597, trouvée 657,1595.

**1.3. 1,2,3,4-tétra-*O*-benzoyl-5-*C*-([1,2,4]triazolo[4,3-a]pyrimidin-3-yl)-α/β-D-xylopyranose (4a)**

**1,2,3,4-tétra-*O*-benzoyl-5-C-([1,2,4]triazolo[1,5-a]pyrimidin-3-yl)-α/β-D-xylopyranose (4b)**

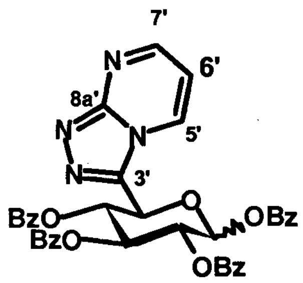

4a

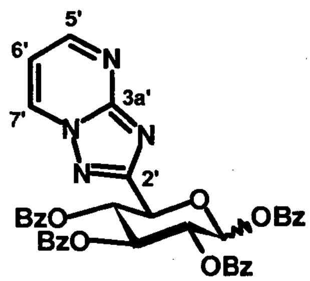

4b

Une solution de 1,2,3,4-tétra-O-benzoyl-5-*C*-(tétrazol-5-yl)-α/β-D-xylopyranose (2) (3,0 g, 4,73 mmol) et de 2-chloropyrimidine (813 mg, 7,10 mmol) dans de la pyridine (38 mL) est maintenue à reflux sous agitation pendant une nuit. Le solvant est éliminé sous pression réduite et le résidu purifié par chromatographie sur gel de silice (cyclohexane-acétate d'éthyle 3:7) pour donner le mélange d'isomères 1,2,3,4-tétra-*O*-benzoyl-5-*C*-([1,2,4]triazolo[4,3-*a*]pyrimidin-3-yl)-α/β-D-xylopyranose (4a) (1,8 g) et 1,2,3,4-tétra-*O*-benzoyl-5-*C*-([1,2,4]triazolo[4,3-a]pyrimidin-3-yl)-α/β-D-xylopyranose (4b) (0,6 g) sous forme de cristaux jaune pâle. Le spectre de RMN du proton dans $CDCl_3$ montrent un rapport α: β de 2:1 pour l'isomère 1,2,3,4-tétra-O-benzoyl-5-*C*-([1,2,4]triazolo[4,3-*a*]pyrimidin-3-yl)-α/β-D-xylopyranose (4a) et de 7:3 pour 1,2,3,4-tétra-*O*-benzoyl-5-*C*-([1,2,4]triazolo[1,5-a]pyrimidin-3-yl)-α/β-D-xylopyranose (4b).
Le rendement global de la réaction est de 74%.

<u>Analyses de 1,2,3,4-tétra-*O*-benzoyl-5-C-([1,2,4]triazolo[4,3-a]pyrimidin-3-yl)-α/β-D-xylopyranose *4a*</u>

Rf = 0,55 (cyclohexane-acétate d'éthyle 1:4)
Point de fusion : 175-179°C
RMN $^1$H (400 MHz, $CDCl_3$) :δ 8,93 (m, 1Hα, H-5'α ou H-7'α), 8,87 (m, 1Hβ, H-5'β ou H-7'β), 8,73 (m, 1Hα, H-5'α ou H-7'α), 8,64 (m, 1Hβ, H-5'β ou H-7'β), 8,22-7,25 (m, 20Hα+20Hβ, H-*aro*), 7,04 (d, 1Hα, *J* 3,5 Hz, H-1α), 7,00 (dd, 1Hα, *J*

4,0 Hz, *J* 7.0 Hz, H-6'α), 6,93 (dd, 1Hβ, *J* 3,5 Hz, *J* 6,5 Hz, H-6'β), 6,57 (t, 1Hα, *J* 9,5 Hz, H-3α), 6,52 (d, 1Hβ, *J* 7,5 Hz, H-1β), 6,29 (m, 2Hβ, H-3β, H-4β), 6,11-6,01 (m, 2Hα+1Hβ, H-4α, H-5α, H-4β), 5,93 (dd, 1Hα, *J* 3,5 Hz, *J* 10,0 Hz, H-2α), 5,90-5,86 (m, 1Hβ, H-5β).
RMN $^{13}C$ (100 MHz, $CDCl_3$) : δ 165,5, 165,2, 165,1, (C=O), 164,6 (C-3'β), 164,4 (C-3'α), 155,1 (C-8a'α, C-8a'β), 154,2 (C-5'α ou C-7'α), 154,0 (C-5'β ou C-7'β), 140,3 (C-5'β ou C-7'β), 140,1 (C-5'α ou C-7'α), 134,4, 134,2, 133,8, 133,7, 133,5, 132,5, 132,4, 130,2-128,3 (C-*aro*), 110,4 (C-6'α, C-6'β), 93,0 (C-1β), 89,7 (C-1α), 71,5 (C-2β ou C-3β ou C-4β ou C-5β) 70,6 (C-2β ou C-3β ou C-4β ou C-5β), 70,4 (C-2α), 70,0 (C-2β ou C-3β ou C-4β ou C-5β), 69,5 (C-3α ou C-4α), 69,1 (C-3α ou C-4α), 68,7 (C-5β).

Masse calculée pour $C_{38}H_{29}N_4O_9$ $[M+H]^+$ : 685,1935, trouvée 685,1953

Analyses de 1,2,3,4-tétra-*O*-benzoyl-5-*C*-([1,2,4]triazolo[1,5-a]pyrimidin-3-yl)-α/β-D-xylopyranose *4b*

Rf = 0,65 (cyclohexane-acétate d'éthyle 1:4)
Point de fusion : 188-190 °C
RMN $^1H$ (400 MHz, $CDCl_3$) : δ 8,80-8,71 (m, 2Hα + 2Hβ, H-5'α, H-7'α, H-5'β, H-7'β), 8,22-7,30 (m, 20Hα + 20Hβ, H-*aro*), 7,10 (dd, 1Hα, *J* 6,5 Hz, *J* 4,5 Hz, H-6'α), 7,07-7,02 (m, 1Hα + 1Hβ, H-6'β, H-1α), 6,47 (m, 1Hα + 1Hβ, H-1β + H-3α), 6,27-6,16 (m, 1Hα + 2Hβ, H-4α, H-3β, H-4β), 6,06 (m, 1Hβ, H-4β), 5,89 (dd, 1Hα, *J* 3,5 Hz, *J* 10,0 Hz, H-2α), 5,73 (d, 1Hα, *J* 10,0 Hz, H-5α), 5,53 (d, 1Hβ, *J* 9,0 Hz, H-5β).
RMN $^{13}C$ (100 MHz, $CDCl_3$) : δ 165,8, 165,6, 165,3, 165,0, 164,8, 164,5, 164,2 (C=O), 164,0 (C-2'α), 163,8 (C-2'β), 155,3 (C-3a'α, C-3a'β), 155,1 (C-7'α), 155,0 (C-7'β), 136,0 (C-5'α, C-5'β), 134,0, 133,7, 133,4-133,2, 130,2-128,2 (C-*aro*), 110,7 (C-6'α), 110,6 (C-6'β), 92,9 (C-1β), 90,0 (C-1α), 72,5 (C-3β ou C-4β ou C-5β), 72,0 (C-3β ou C-4β ou C-5β), 71,3 (C-4α), 70,6 (C-3α), 70,3 (C-2α), 69,4 (C-5α).
Masse calculée pour $C_{38}H_{29}N_4O_9$ $[M+H]^+$ : 685,2524, trouvée : 685,1953

**1.4. 2,3,4-tri-*O*-benzoyl-5-*C*-([1,2,4]triazolo[1,5-a]pyrimidin-2-yl)-α/β-D-xylopyranose (5b)**

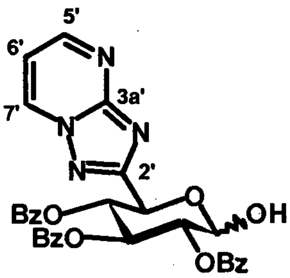

5b

A une solution du composé 1,2,3,4-tétra-*O*-benzoyl-5-*C*-([1,2,4]triazolo[4,3-a]pyrimidin-3-y)-α/β-D-xylopyranose (4a) ou 1,2,3,4-tétra-*O*-benzoyl-5-*C*-([1,2,4]triazolo[1,5-a]pyrimidin-3-y)-α/β-D-xylopyranose (4b) (500 mg, 0,73 mmol) dans du N,Ndiméthylformamide (19 mL) à 0°C, est ajouté de l'acétate d'hydrazine (101 mg, 1,10 mmol) par petites portions en 10 min. Le milieu réactionnel est agité 1 h à 0°C puis 2 h à température ambiante.
Le solvant est éliminé sous pression réduite et le résidu purifié par chromatographie sur gel de silice (cyclohexane-acétate d'éthyle 1:4) pour donner le 2,3,4-tri-*O*-benzoyl-5-*C*-([1,2,4]triazolo[1,5-a]pyrimidin-2-yl)-α/β-D-xylopyranose 5 (284 mg, 67%) sous forme de cristaux blancs. Le spectre de RMN du proton dans $CDCl_3$ montre un rapport α:β de 4:1.
Rf = 0,30 (cyclohexane-acétate d'éthyle 1:4)
Point de fusion : 133-135°C
RMN $^1H$ (400 MHz, $CDCl_3$) pour l'anomère α: δ 8,77 (dd, 1 H, *J* 2,0 Hz, *J* 7,0 Hz, H-5' ou H-7'), 8,69 (m, 1 H, H-5' ou H-7'), 8,02-7,27 (m, 15H, H-*aro*), 7,04 (m, 1 H, H-6'), 6,43 (t, 1 H, *J* 10,0 Hz, H-3), 6,11 (t, 1 H, *J* 10,0 Hz, H-4), 5,95 (d, 1 H, *J* 3,5 Hz, H-1), 5,88 (d, 1 H, *J* 10,0 Hz, H-5), 5,49 (dd, 1 H, *J* 3,5 Hz, *J* 10,0 Hz, H-2).
RMN $^{13}C$ (100 MHz, $CDCl_3$) pour l'anomère α: δ 165,8, 165,7, 165,0 (C=O), 164,9 (C-2'), 155,2 (C-3a'), 155,1 (C-7'), 136,1 (C-5'), 133,2-132,9, 129,3-128,2 (C-*aro*), 110,8 (C-6'), 90,8 (C-1), 72,2 (C-2), 71,7 (C-4), 70,5 (C-3), 66,3 (C-5).
Masse calculée pour $C_{31}H_{25}N_4O_8$ $[M+H]^+$ : 581,1672 ; trouvée 581,1661

**1.5. Trichloroacétimidate de 2,3,4-tri-*O*-benzoyl-5-*C*-([1,2,4]triazolo[1,5-a]pyrimidin-2-yl)-α-D-xylopyranosyle (6)**

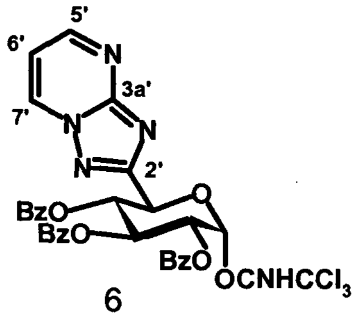

6

A une solution de 2,3,4-tri-*O*-benzoyl-5-*C*-([1,2,4]triazolo[1,5-a]pyrimidin-2-yl)-α/β-D-xylopyranose 5 (650 mg, 1,12 mmol) dans du dichlorométhane (15 mL) à température ambiante, sont ajoutés du 1,8-diazobicyclo[5.4.0]undec-7-ène (186 μL, 1,24 mmol) puis du trichloroacétonitrile (2,2 ml, 21,94 mmol). Le milieu réactionnel est agité 1,5 h à température ambiante. Une solution d'acide acétique (70 μL, 1,22 mmol) dans de l'eau (7 mL) est ajoutée. Les phases sont séparées, la phase organique est lavée par de l'eau (7 mL) puis séchée sur sulfate de sodium.
Le solvant est éliminé sous pression réduite et le résidu purifié par chromatographie sur gel de silice (silice préalablement neutralisée par des lavages avec une solution de triéthylamine, 5% dans de l'acétate d'éthyle) (cyclohexane-acétate d'éthyle 1:1) pour donner le trichloroacétimidate de 2,3,4-tri-*O*-benzoyl-5-*C*-([1,2,4]triazolo[1,5-a]pyrimidin-2-yl)-α-D-xylopyranosyle (6) (565 mg, 70%) sous forme de cristaux jaune.
Rf = 0,35 (cyclohexane-acétate d'éthyle 2:3)
Point de fusion : 72-73°C
$[\alpha]^{25}_D$ = 117,2 (c 0,1, $CDCl_3$)
RMN $^1H$ (400 MHz, $CDCl_3$) : δ 8,81-8,76 (m, 2H, H-5', H-7'), 8,69 (s, 1H, NH), 8,00-7,29 (m, 15H, H-*aro*), 7,11 (dd, 1 H, *J* 4,0 Hz, *J 7,0* Hz, H-6'), 7,00 (d, 1 H, *J* 3,5 Hz, H-1), 6,43 (t, 1 H, *J* 10,0 Hz, H-3), 6,20 (t, 1 H, *J* 10,0 Hz, H-4), 5,81 (dd, 1 H, *J* 3,5 Hz, *J* 10,0 Hz, H-2), 5,71 (d, 1H, *J* 10,0 Hz, H-5).
RMN $^{13}C$ (100 MHz, $CDCl_3$): δ 165,6, 165,3, 164,7 (C=O), 164,0 (C-2'), 160,4 (C=N), 155,4 (C-3a'), 155,1 (C-7'), 135,9 (C-5'), 133,5-133,2, 129,9-128,2 (C-*aro*), 110,7 (C-6'), 93,3 (C-1), 71,1 (C-4), 70,6 (C-2), 70,1 (C-3), 69,4 (C-5).

**1.6. 3-*O*-pivaloyl-*N*-éthoxycarbonylnormorphin-6-yl 2,3,4-tri-*O*-benzoyl-5-*C*-([1,2,4]triazolo[1,5-a]pyrimidin-2-yl)-β-D-xylopyranoside (8)**

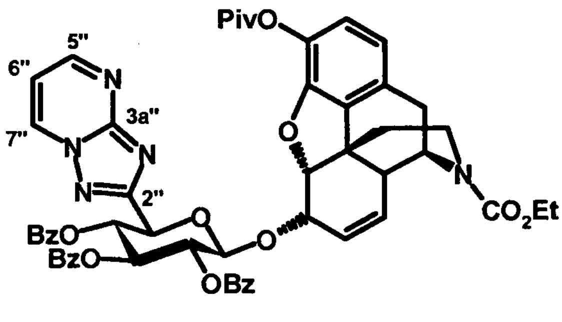

8

A une solution de trichloroacétimidate de 2,3,4-tri-*O*-benzoyl-5-*C*-([1,2,4]triazolo[1,5-a]pyrimidin-2-yl)-α-D-xylopyranosyle (6) (1,5 g, 2,07 mmol) et de 3-O-pivaloyl-N-éthoxycarbonylnormorphine (7) (738 mg, 1,73 mmol) dans du dichlorométhane (24 mL) refroidie à 0°C est ajouté du trifluorométhanesulfonyl de triméthylsilane (1,2 mL, 6,61 mmol). Le milieu réactionnel est agité 30min à 0°C puis 1 h à température ambiante.
De la base de Hünig (1,1 mL) est ajoutée, le mélange est agité 15min puis concentré à sec sous pression réduite. Le résidu est purifié par chromatographie sur gel de silice cyclohexane-acétate d'éthyle 1:4) pour donner le composé

3-*O*-pivaloyl-*N*-éthoxycarbonylnormorphin-6-yl 2,3,4-tri-*O*-benzoyl-5-*C*-([1,2,4]triazolo[1,5-a]pyrimidin-2-yl)-β-D-xylopyranoside (8) (470 mg, 31%) sous forme de cristaux jaune.
Rf = 0,45 (cyclohexane-acétate d'éthyle 1:4)
Point de fusion : 171,5 °C
$[\alpha]^{23}_D$ = - 65,3 (c 0,5, $CDCl_3$)
RMN $^1H$ (400 MHz, $CDCl_3$) : δ 8,80 (dd, 1 H, *J* 2,0 Hz, 4,0 Hz, H-5" ou H-7"), 8,75 (dd, 1 H, *J* 2,0 Hz, 7,0 Hz, H-5" ou H-7"), 8,02-7,29 (m, 15H, H-*aro*), 7,10 (dd, 1 H, *J* 4,0 Hz, *J* 7,0 Hz, H-6"), 6,71 (d, 1 H, *J* 8,0 Hz, H-1), 6,52 (d, 1 H, *J* 8,0 Hz, H-2), 6,21 (m, 1 H, H-4'), 5,95 (t, 1 H, *J* 9,0 Hz, H-3'), 5,75-5,69 (m, 2H, H-2', H-8), 5,47 (d, 1 H, *J* 7,0 Hz, H-1'), 5,34 (d, 1 H, J 9,5 Hz, H-5'), 5,23 (m, 1 H, H-7), 4,98 (d, 1 H, J 5,5 Hz, H-5), 4,93 (m, 1 H, H-9), 4,46 (m, 1H, H-6), 4,20-4,05 (m, 2H, $OCH_2CH_3$), 3,99 (m, 1 H, H-16a), 3,00 (m, 1 H, H-16b), 2,90-2,70 (m, 2H, H-10), 2,48 (m, 1H, H-14), 1,88 (m, 2H, H-15), 1,31-1,25 (m, 12H, $C(CH_3)_3$, $OCH_2CH_3$).
RMN $^{13}C$ (100 MHz, $CDCl_3$): δ 165,7, 165,2, 164,7 (C=O), 155,4 (C-3a"), 154,9 (C-7"), 136,0 (C-5"), 133,1-128,2 (C-*aro*), 122,2 (C-1), 119,2 (C-2), 110,6 (C-6"), 99,4 (C-1'), 89,9 (C-5), 73,0 (C-6), 72,7 (C-3'), 72,2 (C-2'), 71,3 (C-4', C-5'), 61,5 ($OCH_2CH_3$), 49,8 (C-9), 44,3 (C-13), 39,8 (C-14), 37,2 (C-16), 35,3 (C-15), 30,0 (C-10), 27,2 ($C(CH_3)_3$), 14,7 ($OCH_2CH_3$).
Masse calculée pour $C_{55}H_{52}N_5O_{13}$ $[M+H]^+$ : 990,356, trouvée : 990,3596

**1.7. morphin-6-yl 5-C-(5-hydroxy-4,5,6,7-tétrahydro[1,2,4]triazolo[1,5-a]pyrimidin-2-yl)-β-D-xylopyranoside (9)**

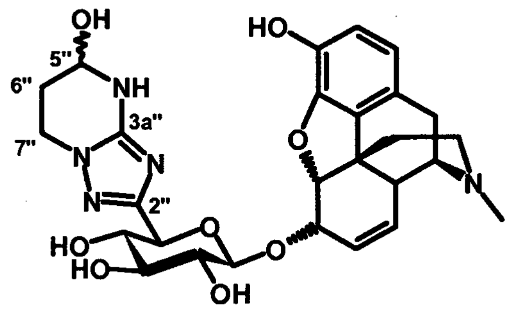

9

A une suspension d'hydrure de lithium et d'aluminium (140 mg, 3,69 mmol) dans du tétrahydrofurane (6 mL), est ajoutée une solution du composé 3-*O*-pivaloyl-*N*-éthoxycarbonylnormorphin-6-yl 2,3,4-tri-*O*-benzoyl-5-*C*-([1,2,4]triazolo[1,5-*a*]pyrimidin-2-yl)-β-D-xylopyranoside (8) (250 mg, 0,28 mmol) dans du tétrahydrofurane (6 mL).
Le milieu réactionnel est agité pendant 1 h à reflux. De l'acétate d'éthyle est ajouté pour détruire l'excès d'hydrure de lithium et d'aluminium et le milieu est porté à pH 1 par ajout d'une solution d'acide chlorhydrique 1 N. Le milieu réactionnel est concentré à sec. Le résidu est purifié une première fois sur colonne de chromatographie en phase inverse ($H_2O$ pure puis ($H_2O$+0,1% acide trifluoroacétique)-acétonitrile 80:20) pour éliminer les sels.
Une deuxième purification par chromatographie préparative en phase inverse (gradient ($H_2O$+0,1% acide trifluoroacétique)- acétonitrile de 95:5 à 20:80) permet d'obtenir le morphin-6-yl 5-*C*-(5-hydroxy-4,5,6,7-tétrahydro[1,2,4]triazolo [1,5-a]pyrimidin-2-yl)-β-D-xylopyranoside (9) sous forme de cristaux blancs (61 mg, 40%).
Point de fusion : 201 °C
$[\alpha]^{23}_D$ = -118,4 (c 0,5,méthanol)
RMN $^1H$ (300 MHz, $D_2O$) : 6,80 (d, 1H, *J* 8,0 Hz, H-1), 6,72 (d, 1 H, *J* 8,0 Hz, H-2), 5,83 (m, 1 H, H-8), 5,43 (m, 1 H, H-7), 5,36 (m, 1 H, H-5"), 5,29 (d, 1 H, *J* 5.5 Hz, H-5), 4,91 (d, 1 H, *J* 8,0 Hz, H-1'), 4,52 (m, 1 H, H-6), 4,51 (d, 1 H, *J* 9,5 Hz, H-5'), 4,32-4,20 (m, 2H, H-9, H-6"a), 4,10 (m, 1 H, H-6"b), 3,78 (t, 1 H, *J* 9,5 Hz, H-4'), 3,68 (t, 1 H, *J* 9,5 Hz, H-3'), 3,53 (dd, 1 H, *J* 8,0 Hz, *J* 9,5 Hz, H-2'), 3,40 (m, 1 H, H-16a), 3,25 (m, 1 H, H-10a), 3,12 (m, 1 H, H-16b), 3,05-2,89 (m, 5H, H-10b, H-14, $NCH_3$), 2,35-2,11 (m, 4H, H-15, H-7").
RMN $^{13}C$ (75 MHz, $D_2O$) :131,2 (C-8), 126,1 (C-7), 120.5 (C-2), 117,8 (C-1), 102,5 (C-1'), 88,3 (C-5), 74,9 (C-3'), 73,7 (C-6), 72,9 (C-2'), 71,7 (C-4'), 71,1 (C-5"), 70,4 (C-5'), 60,6 (C-9), 47,2 (C-16), 40,9 ($NCH_3$), 40,1 (C-6"), 38,5 (C-14), 32,4 (C-15), 26,2 (C-7"), 20,9 (C-10).
Masse calculée pour $C_{27}H_{34}N_5O_8$ $[M+H]^+$ : 556,2407, trouvée : 556,3278.

## Activité biologique

Les composés selon l'invention ont fait l'objet d'essais pharmacologiques permettant de déterminer leur effet analgésique.

Des essais consistant à mesurer l'activité *in vivo* des composés de l'invention sur une réponse réflexe nociceptive ont été effectués. Dans cette approche, la latence de la réponse réflexe nociceptive de l'animal est mesurée comme témoin de la douleur.

### Test du « Tail-Flick »

## Mode opératoire

L'activité analgésique a été déterminée par le test du « Tail- Flick » chez la souris male Swiss (Iffa Credo). Ce test est basé sur le réflexe nociceptif spontané de retrait de la queue de l'animal provoqué par un stimulus thermique douloureux (source infra-rouge). Le test du « Tail-flick » (test de D'Amour et Smith, 1941, Pharmacol Exp Ther;72 : 74-79) consiste, après administration d'un produit, à placer la queue d'une souris au point focal de la source infrarouge de façon à produire un stimulus thermique nociceptif (température de surface d'environ 55-60°C). Le temps de réaction (TR) de la souris (latence entre le moment où le faisceau lumineux est déclenché et le moment où la souris retire sa queue) a été mesuré en duplicate à différents temps allant de 20 minutes à 120 minutes après l'administration du produit. L'intensité de chaleur est réglée de façon à ce que ce réflexe de retrait soit compris entre 0,5 et 3,5 secondes chez des animaux témoins et représente arbitrairement le critère pour une analgésie minimale (0 %). Deux mesures de temps de réaction ont été réalisées avant administration du produit pour chaque souris et permettent d'établir un temps de mesure de base. Un temps maximum de 8 secondes a été choisi comme temps maximum de réaction de manière à ne pas induire de dommage tissulaire par brulure chez les animaux et représente arbitrairement le critère pour une analgésie maximale (100 %). Le temps de réaction est augmenté par les analgésiques par rapport à un animal témoin ne recevant pas de traitement. Les produits ont été administrés par voie sous-cutanée et orale à des doses comprises entre 1,25 et 30 mg/kg.

## Résultats

Les résultats obtenus pour les composés de l'invention sont représentés par les données suivantes :

- le pourcentage d'activité analgésique maximale (index % MPE max) obtenue pour chaque composé (à une dose testée),
- la $DE_{50}$ (exprimée en mg/kg) correspondant à la dose efficace pour chaque composé pour laquelle une analgésie de 50 % à été obtenue ; celle-ci est calculée à un temps donné après l'administration des composés ; et
- la durée de l'action analgésique à une dose donnée.

Le pourcentage d'activité analgésique (% MPE) est déterminé par la formule suivante :

**% MPE = (TRpost-administration - TRpré-administration)*100/(TRmax - TR pré-administration).**

Parmi les composés les plus actifs dont l'activité a été évaluée par voie sous cutanée, à titre d'exemple le morphin-6-yl 5-C-(5-hydroxy-4,5,6,7-tétrahydro[1,2,4]triazolo[1,5-*a*]pyrimidin-2-yl)-β-D-xylopyranoside présente un pourcentage d'activité analgésique maximale (index % MPE max) de 80% pour la dose de 1,25 mg/kg et une $DE_{50}$ inférieure à 1,25 mg/kg déterminée 60 minutes après administration. Les effets analgésiques sont persistants et d'une durée supérieure à 120 minutes par cette voie d'administration.

Il apparaît donc que les composés selon l'invention ont une activité analgésique.

Les composés selon l'invention peuvent donc être utilisés pour la préparation de médicaments, en particulier de médicaments destinés au traitement ou à la prévention de la douleur.

Ainsi, selon un autre de ses aspects, l'invention a pour objet des médicaments qui comprennent un composé de formule (I), ou un sel d'addition de ce dernier à un acide pharmaceutiquement acceptable, ou encore un hydrate ou un solvate.

Ces médicaments trouvent leur emploi en thérapeutique, notamment dans le traitement et la prévention de la douleur aiguë ou chronique notamment périphérique ou associée à des maladies inflammatoires telles que l'arthrite, l'arthrite rhumatoïde, l'ostéoarthrite, la spondylite, la goutte, la vascularite, la maladie de Crohn et le syndrome du colon

irritable, de douleurs neuropathiques, musculaires, osseuses, post-opératoires, de la migraine, les lombalgies, les douleurs associées aux cancers, au diabète au bien encore au SIDA.

Les composés selon l'invention, en tant qu'antalgiques, trouvent encore leur emploi dans le traitement des dysfonctions sexuelles et en particulier dans le traitement de l'éjaculation précoce chez l'homme.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, intraoculaire, le principe actif de formule (I) ci-dessus, ou son sel, solvate ou hydrate éventuel peut être administré sous forme unitaire d'administration, en mélange avec des excipients pharmaceutiques classiques, aux animaux et aux êtres humains pour le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

## Revendications

1. Composé de formule générale (I)

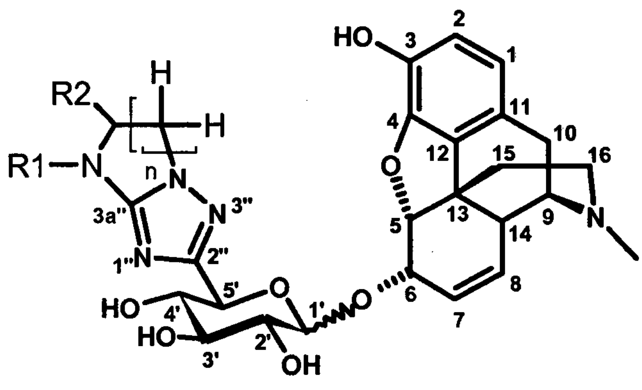

(I)

dans laquelle :

R1 représente un atome d'hydrogène ou un groupe $(C_1\text{-}C_4)$alkyle,
R2 représente un groupe hydroxyle, un groupe thiol, un groupe $(C_1\text{-}C_4)$alkyloxy ou un groupe thio$(C_1\text{-}C_4)$alkyle, et

n est un nombre entier égal à 1 ou 2,

à l'état de base ou de sel d'addition à un acide ainsi qu'à l'état d'hydrate ou de solvate.

**2.** Composé de formule générale (I) selon la revendication 1, **caractérisé en ce que** ledit composé présente une ou plusieurs des caractéristiques suivantes :

- R1 est un atome d'hydrogène,
- R2 est un groupe hydroxy ; et
- n est égal à 2.

**3.** Composé de formule générale (I) selon la revendication 1 ou 2, **caractérisé en ce que** ledit composé est le morphin-6-yl 5-*C*-(5-hydroxy-4,5,6,7-tétrahydro[1,2,4]triazolo[1,5-a]pyrimidin-2-yl)-β-D-xylopyranoside.

**4.** Médicament, **caractérisé en ce qu'**il comprend un composé de formule générale (I) selon l'une quelconque des revendications 1 à 3, ou un sel d'addition de ce composé à un acide pharmaceutiquement acceptable, ou encore un hydrate ou un solvate du composé de formule (I).

**5.** Composition pharmaceutique, **caractérisée en ce qu'**elle comprend un composé de formule générale (I) selon l'une quelconque des revendications 1 à 3, ou un sel pharmaceutiquement acceptable, un hydrate ou un solvate de ce composé, ainsi qu'au moins un excipient pharmaceutiquement acceptable.

**6.** Utilisation d'un composé de formule générale (I) selon l'une quelconque des revendications 1 à 3 pour la préparation d'un médicament destiné au traitement ou à la prévention de la douleur.

## Patentansprüche

**1.** Verbindung der allgemeinen Formel (I)

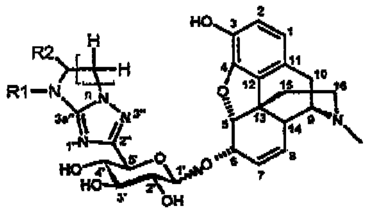

(I)

worin:

R1 für ein Wasscrstoffatom oder eine ($C_1$-$C_4$)-Alkylgruppe steht,
R2 für eine Hydroxylgruppe, eine Thiolgruppc, eine ($C_1$-$C_4$)-Alkyloxygruppe oder eine Thio-($C_1$-$C_4$)-alkylgruppe steht, und
n eine ganze Zahl gleich 1 oder 2 ist,

im Zustand der Base oder des Additionssalizes mit einer Säure sowie im Zustand des hydrats oder Solvats.

**2.** Verbindung der allgemeinen Formel (I) nach Anspruch 1, **dadurch gekennzeichnet, dass** diese Verbindung eines oder mehrere der folgenden Merkmale aufweist:

- R1 ist ein Wasserstoffatom,
- R2 ist eine Hydroxygruppe und
- n ist gleich 2.

**3.** Verbindung der allgemeinen Formel (I) nach Anspruch 1 oder 2, **dadurch gekennzeichnet, dass** es sich bei der Verbindung um Morphin-6-yl-5-*C*-(5-hydroxy-4,5,6,7-tetrahydro[1,2,4]triazolo[1,5-*a*]pyrimidin-2-yl)-β-D-xylopyranosid handelt.

4. Arzneimittel, **dadurch gekennzeichnet, dass** es eine Verbindung der allgemeinen Formel (I) nach einem der Ansprüche 1 bis 3 oder ein Additionssalz dieser Verbindung mit einer pharmazeutisch annehmbaren Säure oder auch ein Hydrat oder ein SolvaL der Verbindung der Formel (I) umfasst.

5. Pharmazeutische Zusammensetzung, **dadurch gekennzeichnet, dass** sie eine Verbindung der allgemeinen Formel (I) nach einem der Ansprüche 1 bis 3 oder ein pharmazeutisch annehmbares Salz, ein Hydrat oder ein Solvat dieser Verbindung sowie mindestens einen pharmazeurisch annehmbaren Excipienten umfasst.

6. Verwendung einer Verbindung der allgemeinen Formel (I) nach einem der Ansprüche 1 bis 3 zur Herstellung eines Arzneimittels für die Behandlung oder Vorbeugung von Schmerz.

## Claims

1. Compound of general formule (T)

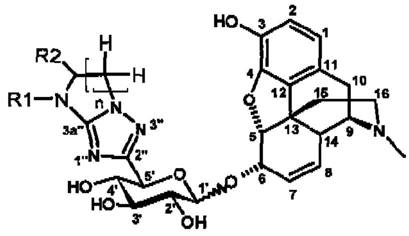

(I)

in which:

R1 represents a hydrogen atom or a group ($C_1$-$C_4$)alkyl,
R2 represents a hydroxyl group, a Lhiol group, a group ($C_1$-$C_4$)alkyloxy or a group thio($C_1$-$C_4$)alkyl, and
n is an integer equal to 1 or 2,

in the form of base or of acid-addition salt, and also in the form of hydrate or solvale.

2. Compound of general formula (I) according to Claim 1, **characterized in that** the said compound has one or more of the following characteristics:

- R1 is a hydrogen atom,
- R2 is a hydroxyl group; and
- n is equal to 2. ,

3. Compound of general formula (I) according to Claim 1 or 2, **characterized in that** the said compound is morphin-6-yl 5-*C*-(5-hydroxy-4,5,6,7-tetrahydro[1,2,4] triazolo[1,5-a]pyrimidin-2-yl)-β-D-xylcopyranoside.

4. Medicament, **characterized in that** it comprises a compound of general formula (T) according to any one of Claims 1 to 3, or an addition salt of this compound with a pharmaceutically acceptable acid, or alternatively a hydrate or a solvate of the compound of formula (I).

5. Pharmaceutical composition, **characterized in that** it comprises a compound of general formula (I) according to any one of Claims 1 to 3, or a pharmaceutically acceptable salt, a hydrate or a solvate of this compound, and also at least one pharmaceutically acceptable excipient.

6. Use of a compound of general formula (I) according to any one of Claims 1 to 3, for Lhe preparation of a medicament for treating or preventing pain.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9844618 A
- FR 2864082


### Littérature non-brevet citée dans la description


- **GREEN et al.** Protective Groups in Organic Synthesis. John Wiley & Sons, Inc,
- **J. MARCH.** Advances in Organic Chemistry. Wiley Interscience, 310-316
- **PORTOGHESE.** *J. Med. Chem.,* 1972, vol. 15, 208-210
- **D'AMOUR ; SMITH.** *Pharmacol Exp Ther,* 1941, vol. 72, 74-79